(19) Europäisches Patentamt
European Patent Office
Office européen des brevets

(11) Numéro de publication : **0 420 761 B1**

(12) **FASCICULE DE BREVET EUROPEEN**

(45) Date de publication du fascicule du brevet : **13.10.93 Bulletin 93/41**

(21) Numéro de dépôt : **90402686.1**

(22) Date de dépôt : **28.09.90**

(51) Int. Cl.[5] : **C07C 271/12,** C07C 269/04, C11D 1/50, A61K 7/00, B01F 17/00

# (54) Alkylcarbamates de triglycérol, leur préparation et leur application notamment comme agents émulsionnants dans des compositions cosmétiques sous forme de microdispersions de cires.

(30) Priorité : **29.09.89 FR 8912750**

(43) Date de publication de la demande : **03.04.91 Bulletin 91/14**

(45) Mention de la délivrance du brevet : **13.10.93 Bulletin 93/41**

(84) Etats contractants désignés : **AT BE CH DE DK ES GB GR IT LI NL SE**

(56) Documents cités :
**DE-A- 1 617 073**
**DE-C- 1 250 808**
**FR-A- 2 053 631**
**BARTON AND OLLIS, "COMPREHENSIVE ORGANIC CHEMISTRY", VOL. 2, PART 9: "CARBOXYLIC ACIDS AND RELATED COMPOUNDS", 1979, PERGAMON PRESS, LTD, PAGE 1070**

(56) Documents cités :
**BARTON AND OLLIS, "COMPREHENSIVE ORGANIC CHEMISTRY", VOL. 2, PART9: "CARBOXYLIC ACIDS AND RELATED COMPOUNDS", 1979, PERGAMON PRESS, LTD, PAGE 1084**
**BARTON AND OLLIS, "COMPREHENSIVE ORGANIC CHEMISTRY", VOL. 1, PART 4: "ALCOHOLS, PHENOLS, ETHERS, AND RELATED COMPOUNDS", 1979, PERGAMON PRESS, LTD, PAGES 676-678**

(73) Titulaire : **L'OREAL**
**14, Rue Royale**
**F-75008 Paris (FR)**

(72) Inventeur : **Zysman, Alexandre**
**6, rue George Eastman**
**F-75013 Paris (FR)**
Inventeur : **Sebag, Henri**
**26, rue Erlanger**
**F-75016 Paris (FR)**

(74) Mandataire : **Tonnellier, Jean-Claude et al**
**Cabinet Nony & Cie. 29, rue Cambacérès**
**F-75008 Paris (FR)**

**EP 0 420 761 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet de nouveaux esters de polyols, leur préparation et leur application, notamment comme agents tensioactifs.

Dans le brevet français n° 84.11687 (2.549.826) on a décrit des monoéthers d'alcools gras et de triglycérol, utilisables notamment comme agents émulsionnants dans des compositions cosmetiques, et leur préparation sous la forme de composés bien définis au départ du dérivé bis-isopropylidène du triglycérol linéaire.

On a maintenant découvert que certains alkylcarbamates de triglycérol présentent des propriétés tensioactives intéressantes, qui permettent de les utiliser notamment dans la préparation de compositions cosmétiques.

On sait par ailleurs qu'il est possible d'obtenir avec certaines cires des microdispersions stables et diluables à l'eau indéfiniment, sans agrégation ni sédimentation des particules en suspension. Les microdispersions de cires sont obtenues par fusion de la cire en présence d'un tensioactif, et éventuellement d'une partie de l'eau, puis addition progressive d'eau chaude avec agitation. On observe la formation intermédiaire d'une émulsion du type eau-dans-l'huile, suivie d'une inversion de phase avec obtention finale d'une émulsion du type huile-dans-l'eau. Au refroidissement, on obtient une microdispersion stable de particules colloïdales solides de cire voir par exemple "Microemulsions Theory and Practice", L.M. Prince Ed., Academic Press (1977) pages 21-32.

Les recherches effectuées par le déposant ont montré que les monoéthers de triglycérol du brevet français cité ci-dessus ne permettent pas d'obtenir une microdispersion de cire.

En outre, on a découvert que les alkylcarbamates de triglycérol de l'invention permettent la réalisation de'microdispersions de cires.

De telles microdispersions de cires sont utilisables notamment comme supports de compositions cosmétiques pour cheveux. Cette utilisation fait l'objet de la demande de brevet luxembourgeoise n° 87.457 déposée par la demanderesse le 24 Février 1989 et intitulée : "Utilisation, comme composition cosmétique pour cheveux, d'une microdispersion de cire, et procédé de traitement des cheveux avec une telle composition".

On a découvert en outre que les alkylcarbamates de triglycérol présentent, par rapport aux éthers du brevet français précité, une meilleure tolérance cutanée.

La présente invention a donc pour objet les alkylcarbamates de formule générale suivante :

$$R\text{-}NHCOOCH(CH_2OCH_2CHOHCH_2OH)_2 \qquad (I)$$

dans laquelle :

R représente un groupement alkyle, éventuellement insaturé ayant 10 à 20 atomes de carbone.

Dans les composés de formule I, le groupement R peut représenter notamment un groupement n-décyle, n-dodécyle, n-tétradécyle, n-hexadécyle, n-octadécyle, n-octadécényle (oléyle), 2-hexyldécyle, 2-octyldodécyle, 2-heptylundécyle, etc.

L'invention a également pour objet un procédé de préparation des composés de formule I.

Ce procédé est caractérisé par le fait que l'on utilise comme produit de départ un composé de formule générale :

$$R_1CH_2CH(R_2)CH_2OCH_2CH(OH)CH_2OCH_2CH(R_2)CH_2R_1 \qquad (II)$$

dans laquelle les groupements $R_1$ et $R_2$ pris ensemble forment un groupement divalent de formule :

$$\text{-}O\text{-}CR_3(R_4)\text{-}O\text{-}$$

$R_3$ et $R_4$, identiques ou différents, représentant un radical alkyle inférieur,

le groupement hydroxyle du composé de formule II étant activé sous la forme d'imidazolide ou de chloroformiate,

que l'on soumet ledit composé II a groupement hydroxyle activé à l'action d'une amine $RNH_2$, dans laquelle R est défini comme précédemment, pour obtenir un composé de formule :

$$R_1CH_2CH(R_2)CH_2OCH_2CH(OR_5)CH_2OCH_2CH(R_2)CH_2R_1 \qquad (III)$$

dans laquelle $R_5$ représente un groupement -CO-NHR, et que l'on hydrolyse le composé III pour obtenir le composé I correspondant.

Le composé II activé répond a la formule IIA :

$$R_1CH_2CH(R_2)CH_2OCH_2CH(OR_5)CH_2OCH_2CH(R_2)CH_2R_1 \qquad (IIA)$$

dans laquelle $R_5$ représente un groupement de formule :

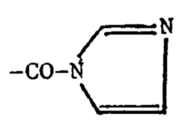

ou un groupement -CO-Cl,

Le composé IIA (imidazolide) peut être obtenu par réaction du composé II (non activé) sur le carbonyldiimidazole.

La réaction du carbonyldiimidazole et du composé de formule II est effectuée en dissolvant ces réactifs dans des solvants tels que le tétrahydrofuranne ou le dichlorométhane. On agite pendant quelques heures à une température comprise entre 20°C et la température d'ébullition du solvant. L'imidazolide de formule IIA obtenu peut être utilisé directement pour la réaction ultérieure.

A partir du composé II, il est également possible de préparer selon les méthodes connues, le chloroformiate correspondant, de formule IIA, avec $R_6$ = -COCl.

Pour la réaction avec l'amine, le composé de formule IIA est dissous dans un solvant tel que le dichlorométhane, puis on ajoute l'amine $RNH_2$ sous la forme d'une solution, par exemple dans le dichlorométhane. Dans le cas où le composé IIA est un chloroformiate, on opère en présence d'un accepteur d'acide chlorhydrique, tel que la pyridine ou la triéthylamine. On agite le milieu réactionnel à une température comprise par exemple entre 20°C et la température d'ébullition du solvant, pendant plusieurs heures. La phase organique est alors lavée à plusieurs reprises avec de l'eau, puis séchée, par exemple sur sulfate de sodium. Le solvant est éliminé par évaporation sous pression réduite. Le composé obtenu, de formule III peut être soumis directement à l'hydrolyse.

L'hydrolyse est effectuée par exemple dans un solvant tel que le méthanol, l'éthanol ou le tétrahydrofuranne en présence d'une petite quantité d'eau et d'un acide protoné.

On peut utiliser des acides minéraux tels que l'acide sulfurique, l'acide phosphorique, l'acide chlorhydrique, ou des acides organiques tels que l'acide paratoluène sulfonique. On emploie de préférence l'acide chlorhydrique.

La quantité d'acide peut varier par exemple de 0,05 à 0,2 mole et de préférence de 0,05 à 0,1 mole d'acide par rapport au nombre de moles du composé de formule III.

Les produits de départ utilisés dans le procédé qui vient d'être décrit sont connus ou peuvent être préparés de façon connue. Le diisopropylidène triglycérol (formule II avec $R_3$ = $R_4$ = méthyle) est un produit commercial (SOLVAY).

L'invention a également pour objet l'utilisation des composés de formule I comme agents tensioactifs, et notamment comme agents émulsionnants, dans des compositions cosmétiques ou dans des supports de compositions cosmétiques. Une application particulière intéressante des composés de formule I est leur utilisation comme agents dispersants dans la préparation de microdispersions aqueuses de cires.

Cette utilisation est principalement caractérisée par le fait que l'on chauffe la cire et au moins un composé de formule I à une température supérieure à la température de fusion de la cire et non supérieure à 100°C, éventuellement en présence d'une partie de l'eau, jusqu'à fusion complète de la cire, que l'on ajoute progressivement l'eau, ou le restant de l'eau, portée à une température au moins égale à ladite température, en agitant, jusqu'à formation d'une microdispersion de cire dans une phase continue aqueuse, puis que l'on laisse refroidir jusqu'à la température ambiante.

L'invention concerne également les microdispersions de cires qui peuvent être obtenues par le procédé qui vient d'être indiqué.

Parmi les cires utilisables, on citera notamment les cires ou mélanges de cires ayant un point de fusion finissante supérieur à 60°C et inférieur à 100°C.

L'agent émulsionnant est constitué par au moins un composé de formule I, éventuellement en mélange avec d'autres agents émulsionnants non ioniques ou anioniques.

La microdispersion peut contenir par exemple de 0,1 à 40% en poids de cire, et de 0,01 à 25% en poids d'agent émulsionnant.

De préférence, le rapport pondéral cire/émulsionnant varie de 1 à 30.

Généralement, la microdispersion de cire contient au moins 35% d'eau.

Dans les microdispersions de cires obtenues, les dimensions des particules de cires sont inférieures à 500nm.

Parmi les cires utilisables on citera par exemple la cire de Carnauba, la cire de Candelilla, la cire d'Alfa et leurs mélanges.

On peut également utiliser les cires qui viennent d'être citées, ou leurs mélanges en combinaison avec une autre cire ou un mélange d'autres cires, par exemple avec une cire de paraffine ; la proportion pondérale de cire de Carnauba et/ou de Candelilla, dans de tels mélanges, est de préférence supérieure ou égale à 50%.

Les tensioactifs qui peuvent être utilisés en mélange avec les composés de formule I sont par exemple des tensioactifs anioniques ayant par exemple une balance lipophile-hydrophile (HLB) pouvant aller de 10 à 40. Ce sont notamment des sels d'acides gras (par exemple sels alcalins ou sels organiques tels que les sels d'amines), lesdits acides gras ayant par exemple de 12 à 18 atomes de carbone et pouvant comporter une double liaison comme dans le cas de l'acide oléique; les sels alcalins ou sels de bases organiques des acides

alkyl-sulfuriques et alkyl-sulfoniques ayant 12 à 18 atomes de carbone, des acides alkyl-arylsulfoniques dont la chaîne alkyle contient de 6 à 16 atomes de carbone, le groupement aryle étant par exemple un groupement phényle. Ce sont également les éthers-sulfates, en particulier les produits de sulfatation des alcools gras et alkylphénols polyalcoxylés, dans lesquels la chaîne aliphatique comporte de 6 à 20 atomes de carbone et la chaîne polyalcoxylée de 1 à 30 motifs oxyalkylène, en particulier oxyéthylène, oxypropylène ou oxybutylène.

Les tensioactifs non-ioniques qui peuvent être utilisés en mélange avec les composés de formule I sont notamment les acides gras ou les amides d'acides gras polyalcoxylés et/ou polyglycérolés ; les esters d'acides gras et de polyols polyalcoxylés et/ou polyglycérolés ; les alcools gras ou les alkylphénols polyalcoxylés et/ou polyglycérolés; les alcanediols ou alcènediols-1,2 ou -1,3 polyalcoxylés et/ou polyglycérolés ; et les alkyléthers d'alcanediols ou alcènediols -1,2 ou -1,3 polyalcoxylés et/ou polyglycérolés. Les acides ou alcools gras, éventuellement insaturés, ont par exemple 12 à 24 atomes de carbone, la chaîne alkyle des alkylphénols a par exemple 6 à 16 atomes de carbone, les alcanediols ou alcènediols ont de 9 à 24 atomes de carbone, l'alkyle d'es alkyléthers a de 4 à 20 atomes de carbone, et le nombre de motifs oxyalkylène ou de motifs ($-CH_2CHOH\ CH_2O-$) peut aller de 2 à 40.

Les dérivés non ioniques polyalcoxylés sont notamment des dérivés polyoxyéthylénés, éventuellement polyoxypropylénés.

Les acides gras polyalcoxylés sont des produits commerciaux, notamment les produits vendus sous la dénomination commerciale "Myrj" par la Société ICI AMERICAS.

Les esters d'acides gras et de polyols polyoxyéthylénés pour lesquels le polyol est le sorbitol sont des produits connus commercialisés sous la dénomination commerciale "Tween" par la Société ICI AMERICAS.

Les alcools gras polyoxyéthylénés sont des produits commerciaux, notamment ceux vendus sous la dénomination commerciale "Brij" par la Société ICI AMERICAS.

Les alcools gras polyglycérolés, les alcanediols ou alcènediols polyglycérolés, ou les alkyléthers d'alcanediols ou d'alcènediols polyglycérolés peuvent être préparés par exemple selon les procédés décrits dans les brevets français 1.477.048, 2.025.681, 2.091.516 et 2.465.780, ou selon des procédés analogues.

Les acides gras ou amides d'acides gras polyglycérolés sont notamment décrits dans le brevet français 1.484.723 ou sont encore des produits commerciaux tels que ceux vendus sous les dénominations commerciales de "Plurol" par le Société Gattefosse ou de "Drewpol" par la Société Stefan Company.

L'invention a également pour objet des compositions cosmétiques, notamment des compositions cosmétiques pour cheveux, caractérisées par le fait qu'elles contiennent comme agent tensioactif ou émulsionnant, au moins un composé de formule I.

Les compositions de l'invention contiennent bien entendu tous les ingrédients actifs, véhicules, et ingrédients secondaires usuels que l'on désire y introduire.

Les compositions cosmétiques de l'invention se présentent notamment sous la forme des microdispersions stables de cires qui ont été décrites ci-dessus. Ces compositions sous forme de microdispersions de cires sont utilisables notamment comme lotions coiffantes et aussi comme lotions destinées à améliorer l'aspect des cheveux chez les sujets ayant les cheveux gras, comme cela est décrit dans la demande de brevet luxembourgeoise citée ci-dessus.

Ces compositions, sous forme de microdispersions peuvent contenir un ou plusieurs ingrédients secondaires usuels tels que les agents épaississants, les agents stabilisants, les parfums ou les agents conservateurs.

Ces ingrédients secondaires sont ajoutés selon les cas soit dans les produits de départ (avant préparation de la microdispersion), soit dans la composition terminée.

Les ingrédients hydrosolubles non volatils peuvent être ajoutés éventuellement dans l'eau utilisée pour réaliser la microdispersion.

Les ingrédients liposolubles sont généralement ajoutés à la cire avant la réalisation de la microdispersion.

Ces compositions peuvent être appliquées sur cheveux secs ou mouillés, par exemple avant ou après un shampooing. Elles peuvent être rincées ou non rincées et être appliquées quotidiennement.

Lorsqu'elles sont appliquées avant ou après un shampooing, l'application étant suivie ou non d'un rinçage à l'eau, elles disciplinent les cheveux et communiquent à la coiffure de la tenue et du volume. En outre, elles retardent le phénomène de regraissage des cheveux observé chez les sujets ayant les cheveux gras.

Ces compositions ont un pH qui peut varier de 3 à 10. Le pH peut éventuellement être ajusté à l'aide d'un agent modificateur de pH usuel.

Les exemples suivants illustrent l'invention sans toutefois la limiter :

EXEMPLE 1

Composé de formule I, avec R = hexadécyle.

a) Préparation de l'imidazolide

Dans 500cm3 de dichlorométhane, on solubilise 102g (0,63M) de carbonyldiimidazole. A cette solution, on ajoute 500cm3 de dichlorométhane contenant 200g (0,62M) de diisopropylidène triglycérol commercialisé par la Société SOLVAY. On agite le milieu réactionnel pendant 5 heures à la température ambiante. On le lave ensuite à quatre reprises avec 150cm3 d'eau. On sèche sur sulfate de sodium puis on évapore le solvant sous pression réduite. On obtient 241g d'un composé sous forme d'huile. Sa pureté est suffisante pour l'utilisation directe dans les réactions ultérieures.

b) Réaction avec l'hexadécylamine

Dans 50cm3 de dichlorométhane on solubilise 14,1g (0,034M) de l'imidazolide préparé ci-dessus. A cette solution on ajoute 7,2g (0,03M) d'hexadécylamine solubilisée dans 25cm3 de dichlorométhane. On laisse agiter le milieu réactionnel pendant une nuit à température ambiante. On le lave ensuite à plusieurs reprises avec de l'eau. Après séchage de la phase organique sur sulfate de sodium, suivi de l'évaporation du solvant sous pression réduite, on isole 20g d'un composé cireux.

c) Hydrolyse

Les 20g de composé cireux obtenu ci-dessus sont solubilisés dans 100cm3 de méthanol auxquels on ajoute 2cm3 d'acide chlorhydrique 5N. On laisse agiter le milieu réactionnel durant une nuit. Après filtration d'un léger insoluble, on élimine le solvant sous pression réduite. On obtient 17g d'une cire que l'on recristallise dans un mélange d'heptane et d'acétate d'éthyle (200 : 30). On isole 13g d'une cire blanche. Elle présente un point de Kraft à 0,5% de 21-24°C et un point de trouble >100°C. Elle répond à la formule I, avec R = hexadécyle. Les spectres RMN $^{1}H$ et $^{13}C$ sont en accord avec la structure indiquée.

Analyse élémentaire

| | calculé | trouvé |
|---|---|---|
| C% | 61,51 | 61,2 |
| H% | 10,52 | 10,23 |
| N% | 2,76 | 2,63 |

EXEMPLE 2

Composé de formule I, avec R = docécyle

Dans 100cm3 d'une solution de dichlorométhane contenant 54g (0,13mole) d'imidazolide de diisopropylidène triglycérol préparé selon l'exemple 1(a), on ajoute 19,65g (0,106M) de dodécylamine solubilisée dans 150cm3 du même solvant. On agite le milieu réactionnel à la température ambiante pendant une nuit puis on le lave à trois reprises avec 100cm3 d'eau. On sèche sur sulfate puis on évapore le solvant sous pression réduite. On isole 62g d'une huile visqueuse très légèrement teintée. Dans 300cm3 de méthanol on solubilise 60g du produit huileux obtenu, puis on ajoute 5cm3 d'acide chlorhydrique 5N. On laisse sous agitation à température ambiante pendant une nuit. On évapore le solvant sous pression réduite et on isole 49,2g d'une huile visqueuse.

On soumet 30g de cette huile à une purification sur colonne de silice (Kieselgel 60, Merck) avec comme éluant un mélange de dichlorométhane et de méthanol (90 : 10).

On isole 12g d'un composé de formule I, avec R = docécyle, sous la forme d'une cire blanche.

Le spectre RMN $^{13}C$ est en accord avec la structure indiquée.

EXEMPLE 3

Composé de formule I, avec R = oléyle

Dans 100cm3 d'une solution de dichlorométhane contenant 5g (0,12M) d'imidazolide de diisopropylidène triglycérol préparé selon l'exemple 1(a), on ajoute 26,75g (0,1M) d'oléylamine solubilisée dans 100cm3 du même solvant. On laisse agiter le milieu réactionnel pendant 5 heures puis on élimine le solvant sous pression réduite. On isole 76g d'une huile que l'on soumet à une purification par passage sur un verre fritté rempli de silice Kieselgel 60H (Merck) et en éluant avec un mélange de dichlorométhane et de méthanol (9 : 1).
Après élimination du solvant sous pression réduite, on isole 66g de composé sous forme d'une huile. On soumet 60g de cette huile à une hydrolyse en la solubilisant dans 250cm3 d'éthanol en présence de 30cm3 d'acide chlorhydrique 1N et en chauffant le milieu réactionnel 6 heures à 60°C. Après élimination du solvant sous pression réduite, on soumet la cire obtenue à une purification par passage sur un verre fritté rempli de silice Kieselgel 60H et avec comme éluant un mélange de dichlorométhane et de méthanol (95 : 5).
On isole 40g du composé de formule I, avec R = oléyle, sous la forme d'une cire translucide.
Le spectre RMN $^{13}C$ est en accord avec la structure indiquée.

EXEMPLES DE PREPARATION DE MICRODISPERSIONS DE CIRES (EXEMPLES A à D)

Ces microdispersions de cires sont préparées selon la méthode qui a été décrite dans la description.
La cire utilisée est la cire de Carnauba.
L'agent émulsionnant est un composé de formule I.
Les constituants et leurs proportions ainsi que les caractéristiques physico-chimiques de la microdispersion obtenue (aspect, diamètre moyen des particules de cire, polydispersité) sont résumés dans le tableau suivant :

| Emulsionnant | | | Additif | | Quantité de cire (%) (1) | Caractéristiques physico-chimiques | | |
|---|---|---|---|---|---|---|---|---|
| Exemple | R | Quantité (%)(1) | Nature | Quantité (%)(1) | | Aspect | diamètre moyen | polydispersité |
| A | $C_{16}H_{33}$ | 2,11 | KOH | 0,03 | 10 | liquide jaunâtre opalescent | 110nm | 0,2 |
| B | $C_{12}H_{25}$ | 1,88 | DCP(2) | 0,09 | 10 | liquide blanchâtre | 258nm | 0,13 |
| C | " | " | KOH | 0,03 | 10 | liquide blanchâtre léger reflet opalescent | 208nm | 0,12 |
| D | $C_{18}H_{35}$ | 2,21 | KOH | 0,03 | 10 | liquide jaunâtre opalescent | 71nm | 0,14 |

(1) : % pondéraux par rapport à 100g de dispersion dans l'eau

(2) : DCP = Dicétylphosphate (sel de sodium)

A titre de comparaison on a tenté de préparer une microdispersion de cire dans les mêmes conditions (émulsionnant = 1,93% ; potasse: 0,03% ; cire : 10%) en utilisant comme agent émulsionnant le composé de formule

$$C_{16}H_{33}OCH(CH_2OCH_2CHOHCH_2OH)_2$$

Il n'a pas été possible d'obtenir une microdispersion.

EXEMPLES DE PREPARATION DE COMPOSITIONS COSMETIQUES (CC1 à CC5)

EXEMPLE CC1

On prépare la composition de soins suivante, en incorporant dans la microdispersion de cire les autres constituants, dans l'ordre indiqué :

- Microdispersion de cire obtenue à l'exemple A .................................. 98 g
- Hydroxypropylméthylcellulose vendue par la Société DOW CHEMICAL sous la dénomination METHOCEL F4M.......................... 1,5 g
- p.hydroxybenzoate de méthyle................. 0,2 g
- Dérivé d'imidazolidinylurée vendu sous la dénomination GERMALL 115 par la Société SUTTON LABS......................... 0,3 g

Cette composition est appliquée sur cheveux propres et secs en prenant soin de l'étaler sur toute la longueur du cheveu.

On utilise de 2 à 5 g de composition par tête.

La coiffure obtenue présente du gonflant et est disciplinée.

EXEMPLE CC2

On prépare une composition, avant shampoing, de gel fluide suivante, en incorporant dans la microdispersion de cire les autres constituants, dans l'ordre indiqué :

- Microdispersion de cire obtenue à l'exemple B................................. 20 g
- Acide polyacrylique réticulé (PM : 1 250 000) vendu par la société GOODRICH sous la dénomination CARBOPOL 941........................ 1 g
- Dérivé d'imidazolidinylurée vendu sous la dénomination GERMALL 115 par la société SUTTON LABS....................... 0,3 g
- Hydroxyde de sodium qs pH : 7
- Eau ..................................qsp 100 g

Cette composition est appliquée sur des cheveux secs mais non lavés, en veillant à son bon étalement.

Après un temps de pose de 2 à 3 minutes, on procède au rinçage à l'eau puis au shampoing.

On obtient des cheveux doux, brillants, disciplinés et ayant du volume.

EXEMPLE CC3

On prépare une composition capillaire, en incorporant dans la microdispersion de cire les autres constituants, dans l'ordre indiqué :

- Microdispersion à 10% de cire de Carnauba préparée selon l'exemple C..... 99,65 g
- p.hydroxybenzoate de méthyle............... 0,15 g
- Dérivé d'imidazolidinylurée vendu sous la dénomination GERMALL 115 par la société SUTTON LABS......................... 0,20 g

On utilise cette composition comme dans l'exemple CC1.

La chevelure présente, après application de cette composition, du gonflant. Les cheveux sont disciplinés et doux.

EXEMPLE CC4

On prépare la composition de traitement avant-shampooing suivante, en incorporant dans la microdispersion de cire les autres constituants, dans l'ordre indiqué :

- Microdispersion à 10% de cire de Carnauba selon l'exemple D .............................. 97,5 g
- Hydroxypropylcellulose commercialisée sous la dénomination KLUCEL H par la société HERCULES... 2 g
- Parahydroxybenzoate de méthyle.................. 0,2 g
- Dérivé d'imidazolidinylurée vendu sous la dénomination GERMALL 115 par la société SUTTON LABS................................... 0,3 g

La composition est appliquée dans les conditions de l'exemple CC2.

La chevelure ainsi traitée présente beaucoup de volume. Les cheveux sont brillants et doux.

EXEMPLE CC5

On prépare une composition capillaire ayant la composition suivante, en incorporant dans la microdispersion de cire les autres constituants, dan l'ordre indiqué :

- Microdispersion de cire obtenue à l'exemple C.................................. 10 g
- CARBOPOL 941.................................. 1,5 g
- NaOH.............................................. 0,6 g
- PEG-15 COCAMINE.............................. 3 g
- GERMALL 115.................................. 0,2 g
- p-hydroxybenzoate de méthyle.................. 0,2 g
- Sorbate de potassium.......................... 0,3 g
- Parfum , ........q.s.
- Triéthanolamine, q.s. pH = 7
- Eau...................................q.s.p.. 100 g

PEG-15 COCAMINE : Polyéthylèneglycol amine d'acide de coprah, selon la définition du CTFA (Cosmetic, Toiletry and Fragrance Association); produit vendu par AKZO sous la dénomination commerciale ETHOMEEN C25.

En veillant toujours à un bon étalement, on applique cette composition sur des cheveux secs et propres.

Après traitement, la chevelure présente du volume et de la brillance.

Les cheveux sont disciplinés.

## Revendications

**1.** Composés de formule générale suivante :

$$R\text{-}NHCOOCH(CH_2OCH_2CHOHCH_2OH)_2 \qquad (I)$$

dans laquelle :
R représente un groupement alkyle, éventuellement insaturé ayant 10 à 20 atomes de carbone.

**2.** Composés selon la revendication 1 caractérisés par le fait que R est choisi parmi les groupements n-décyle, n-dodécyle, n-tétradécyle, n-hexadécyle, n-octadécyle, n-octadécényle (oléyle), 2-hexyldécyle, 2-octyldodécyle et 2-heptylundécyle.

**3.** Procédé de préparation des composés de formule I selon les revendications 1 et 2, caractérisé par le fait que l'on utilise comme produit de départ un composé de formule générale :

$$R_1CH_2CH(R_2)CH_2OCH_2CH(OH)CH_2OCH_2CH(R_2)CH_2R_1 \qquad (II)$$

dans laquelle les groupements $R_1$ et $R_2$ pris ensemble forment un groupement divalent de formule :

$$\text{-}O\text{-}CR_3(R_4)\text{-}O\text{-}$$

$R_3$ et $R_4$, identiques ou différents, représentant un radical alkyle inférieur,

le groupement hydroxyle du composé de formule II étant activé sous la forme d'imidazolide ou de chloroformiate,

que l'on soumet ledit composé II à groupement hydroxyle activé à l'action d'une amine $RNH_2$, dans laquelle R est défini comme précédemment, pour obtenir un composé de formule :

$$R_1CH_2CH(R_2)CH_2OCH_2CH(OR_5)CH_2OCH_2CH(R_2)CH_2R_1 \qquad (III)$$

dans laquelle $R_5$ représente un groupement -CO-NHR, et que l'on hydrolyse le composé III pour obtenir le composé I correspondant.

**4.** Utilisation des composés de formule I tels que définis dans l'une quelconque des revendications 1 et 2, comme agents tensioactifs ou émulsionnants.

**5.** Utilisation selon la revendication 4, comme agents tensioactifs ou émulsionnants dans des microdispersions aqueuses de cires.

**6.** Utilisation selon la revendication 5, dans laquelle on utilise une cire choisie parmi la cire de Carnauba, la

cire de Candelilla, la cire d'Alfa, et leurs mélanges.

7. Utilisation selon la revendication 5 ou 6, dans laquelle le rapport pondéral cire/émulsionnant est compris dans une gamme allant de 1 à 30.

8. Utilisation selon l'une quelconque des revendications 4 à 7, comme agents tensioactifs ou émulsionnants dans des compositions cosmétiques.

9. Utilisation selon l'une quelconque des revendications 5 à 7, caractérisée par le fait que, pour obtenir une microdispersion aqueuse de cire, on chauffe la cire et l'émulsionnant à une température supérieure à la température de fusion de la cire et non supérieure à 100°C,
éventuellement en présence d'une partie de l'eau, jusqu'à fusion complète de la cire, on ajoute progressivement l'eau, ou le restant de l'eau portée à une température au moins égale à ladite température, en agitant jusqu'à formation d'une microdispersion de cire dans une phase continue aqueuse, puis on laisse refroidir jusqu'à la température ambiante.

10. Microdispersion de cire, se présentant sous la forme d'une dispersion stable, dans un véhicule liquide aqueux, de particules de dimensions inférieures à 500nm, lesdites particules étant constituées essentiellement d'une cire ou d'un mélange de cires, caractérisée par le fait que ladite microdispersion contient comme agent émulsionnant au moins un composé de formule I tel que défini dans l'une quelconque des revendications 1 et 2.

11. Microdispersion selon la revendication 10, caractérisée par le fait que ladite cire est choisie parmi la cire de Carnauba, la cire de Candelilla, la cire d'Alfa, et leurs mélanges.

12. Microdispersion selon la revendication 10 ou 11, caractérisée par le fait que ledit agent émulsionnant est présent à une concentration de 0,01 a 25% en poids.

13. Microdispersion selon l'une quelconque des revendications 10 à 12, caractérisée par le fait qu'elle continent de 0,1 à 40% en poids de cire.

14. Microdispersion selon l'une quelconque des revendications 10 à 13, caractérisée par le fait que le "rapport pondéral cire/émulsionnant est compris dans une gamme allant de 1 à 30.

15. Composition cosmétique caractérisée par le fait qu'elle contient comme agent tensioactif ou émulsionnant au moins un composé de formule I tel que défini dans l'une quelconque des revendications 1 et 2.

16. Composition selon la revendication 15, caractérisée par le fait qu'elle se présente sous la forme d'une microdispersion de cire.

17. Composition selon la revendication 16, caractérisée par le fait que ladite microdispersion de cire est telle que définie dans l'une quelconque des revendication 10 à 14.

**Patentansprüche**

1. Verbindungen der folgenden allgemeinen Formel:

$$R\text{-}NHCOOCH(CH_2OCH_2CHOHCH_2OH)_2 \qquad (I)$$

in der:
R eine gegebenenfalls ungesättigte Alkylgruppe mit 10 bis 20 Kohlenstoffatomen bedeutet.

2. Verbindungen nach Anspruch 1, dadurch gekennzeichnet, daß R aus n-Decyl-, n-Dodecyl-, n-Tetradecyl-, n-Hexadecyl-, n-Octadecyl-, n-Octa-decenyl-(Oleyl-), 2-Hexyldecyl-, 2-Octyldodecyl- und 2-Heptylundecyl-Gruppen ausgewählt ist.

3. Verfahren zur Herstellung der Verbindungen der Formel I gemäß den Ansprüchen 1 und 2, dadurch gekennzeichnet, daß man als Ausgangsprodukt eine Verbindung der allgemeinen Formel verwendet:

$$R_1CH_2CH(R_2)CH_2OCH_2CH(OH)CH_2OCH_2CH(R_2)CH_2R_1 \qquad (II)$$

in der die Gruppen $R_1$ und $R_2$ zusammen eine zweiwertige Gruppe der Formel:

$$-O-CR_3(R_4)-O-$$

bilden, wobei $R_3$ und $R_4$ gleich oder verschieden sind und einen Niedrigalkyl-Rest bedeuten, die Hydroxylgruppe der Verbindung der Formel II als Immidazolid oder Chlorformiat aktiviert ist, daß man die Verbindung II an der aktivierten Hydroxylgruppe der Einwirkung eines Amins $RNH_2$ aussetzt, in der R die oben angegebene Bedeutung hat, um eine Verbindung der Formel:

$$R_1CH_2CH(R_2)CH_2OCH_2CH(OR_5)CH_2OCH_2CH(R_2)CH_2R_1 \quad (III)$$

zu erhalten, in der $R_5$ eine Gruppe-CO-NHR bedeutet, und die Verbindung III hydrolisiert wird um die entsprechende Verbindung I zu erhalten.

4. Verwendung der Verbindungen der Formel I gemäß den Ansprüchen 1 und 2 als oberflächlichenaktive Mittel oder Emulgatoren.

5. Verwendung nach Anspruch 4 als oberflächenaktive Mittel oder Emulgatoren in wässrigen Mikrodispersionen von Wachsen.

6. Verwendung nach Anspruch 5, wobei man ein Wachs ausgewählt aus Carnaubau-Candelilla-,Alfawachs und deren Gemische verwendet.

7. Verwendung nach Anspruch 5 oder 6, wobei das Gewichtsverhältnis Wachs/ Emulgator im Bereich 1 bis 30 liegt.

8. Verwendung nach einem der Ansprüche 4 bis 7 als oberflächenaktive Mittel oder Emulgatoren in kosmetischen Zubereitungen.

9. Verwendung nach einem der Ansprüche 5 bis 7, dadurch gekennzeichnet, daß man zur Herstellung einer wässrigen Mikrodispersion von Wachs, das Wachs und den Emulgator auf eine Temperatur oberhalb der Schmelztemperatur des Wachses, jedoch nicht oberhalb 100°C erwärmt, gegebenenfalls in Anwesenheit von etwas Wasser bis zum vollständigen Schmelzen des Wachses, daß man in zunehmenden Anteilen Wasser zugibt oder das restliche Wasser, mindestens auf die genannte Temperatur erwärmt, wobei man bis zur Bildung einer Mikrodispersion des Wachses in einer kontinuierlichen wässrigen Phase rührt und anschließend auf Raumtemperatur abkühlen läßt.

10. Mikrodispersion von Wachs als stabile Dispersion in einem flüssigen, wässrigen Träger aus Partikel mit einer Teilchengröße unterhalb 500 nm, wobei diese Partikel im wesentlichen aus Wachs oder einem Wachsgemisch bestehen, dadurch gekennzeichnet, daß die Mikrodispersion als Emulgator mindestens eine Verbindung der Formel I nach einem der Ansprüche 1 und 2 enthält.

11. Mikrodispersion nach Anspruch 10, dadurch gekennzeichnet, daß das Wachs aus Carnaubau,-Candelilla-, Alfawachs oder ihren Gemischen gewählt ist.

12. Mikrodispersion nach Anspruch 10 oder 11, dadurch gekennzeichnet, daß der Emulgator in einer Konzentration von 0,01 bis 25 Gew.-% vorliegt.

13. Mikrodispersion nach einem der Ansprüche 10 bis 12, dadurch gekennzeichnet, daß sie 0,1 bis 40 Gew.-% Wachs enthält.

14. Mikrodispersion nach einem der Ansprüche 10 bis 13, dadurch gekennzeichnet, daß das Gewichtsverhältnis Wachs/Emulgator in einem Bereich von 1 bis 30 liegt.

15. Kosmetische Zubereitung, dadurch gekennzeichnet, daß sie als oberflächenaktives Mittel oder Emulgator mindestens eine Verbindung der Formel I nach einem der Ansprüche 1 und 2 enthält.

16. Zubereitung nach Anspruch 15, dadurch gekennzeichnet, daß sie in Form einer Mikrodispersion von Wachs vorliegt.

17. Zubereitung nach Anspruch 16, dadurch gekennzeichnet, daß die Mikrodispersion von Wachs eine Mikrodispersion nach einem der Ansprüche 10 bis 14 ist.

## Claims

1. Compounds of the following general formula:

$$R\text{-}NHCOOCH(CH_2OCH_2CHOHCH_2OH)_2 \qquad (I)$$

in which:
R represents an optionally unsaturated alkyl group having 10 to 20 carbon atoms.

2. Compounds according to Claim 1, characterised in that R is chosen from n-decyl, n-dodecyl, n-tetradecyl, n-hexadecyl, n-octadecyl, n-octadecenyl (oleoyl), 2-hexyldecyl, 2-octyldodecyl and 2-heptylundecyl groups.

3. Process for the preparation of the compounds of formula I according to Claims 1 and 2, characterised in that there is used, as starting material, a compound of general formula:

$$R_1CH_2CH(R_2)CH_2OCH_2CH(OH)CH_2OCH_2CH(R_2)CH_2R_1 \qquad (II)$$

in which the groups $R_1$ and $R_2$, taken together, form a divalent group of formula:

$$\text{-}O\text{-}CR_3(R_4)\text{-}O\text{-}$$

$R_3$ and $R_4$, which are identical or different, representing a lower alkyl radical,
the hydroxyl group of the compound of formula II being activated in the imidazolide or chloroformate form,
in that the said compound II containing an activated hydroxyl group is subjected to the action of an amine $RNH_2$, in which R is defined as above, to produce a compound of formula:

$$R_1CH_2CH(R_2)CH_2OCH_2CH(OR_5)CH_2OCH_2CH(R_2)CH_2R_1 \qquad (III)$$

in which $R_5$ represents a group -CO-NHR, and in that the compound III is hydrolysed to produce the corresponding compound I.

4. Use of the compounds of formula I as defined in either of Claims 1 or 2 as surface-active or emulsifying agents.

5. Use according to Claim 4, as surface-active or emulsifying agents in aqueous wax microdispersions.

6. Use according to Claim 5, in which a wax is used chosen from carnauba wax, candelilla wax, alfa wax and their mixtures.

7. Use according to Claim 5 or 6, in which the wax/emulsifying agent ratio by weight is in a range from 1 to 30.

8. Use according to any one of Claims 4 to 7, as surface-active or emulsifying agents in cosmetic compositions.

9. Use according to any one of Claims 5 to 7, characterised in that, to produce an aqueous wax microdispersion, the wax and the emulsifying agent are heated at a temperature greater than the melting temperature of the wax and not greater than 100°C, optionally in the presence of part of the water, until the wax is completely melted, the water, or the rest of the water, brought to a temperature at least equal to the said temperature, is progressively added, stirring being carried out until a wax microdispersion in a continuous aqueous phase is formed, and then the mixture is left to cool to room temperature.

10. Wax microdispersion, existing in the form of a stable dispersion, in an aqueous liquid vehicle, of particles with sizes less than 500 nm, the said particles consisting essentially of a wax or a mixture of waxes, characterised in that the said microdispersion contains, as emulsifying agent, at least one compound of formula I as defined in either of Claims 1 or 2.

11. Microdispersion according to Claim 10, characterised in that the said wax is chosen from carnauba wax, candelilla wax, alfa wax and their mixtures.

12. Microdispersion according to Claim 10 or 11, characterised in that the said emulsifying agent is present at a concentration of 0.01 to 25 % by weight.

13. Microdispersion according to any one of Claims 10 to 12, characterised in that it contains from 0.1 to 40 % by weight of wax.

**14.** Microdispersion according to any one of Claims 10 to 13, characterised in that the wax/emulsifying agent ratio by weight is in a range from 1 to 30.

**15.** Cosmetic composition, characterised in that it contains, as surface-active or emulsifying agent, at least one compound of formula I as defined in either of Claims 1 or 2.

**16.** Composition according to Claim 15, characterised in that it exists in the form of a wax microdispersion.

**17.** Composition according to Claim 16, characterised in that the said wax microdispersion is as defined in any one of Claims 10 to 14.